# FASCICULE DE BREVET EUROPEEN

(11) **EP 2 421 633 B1**
(45) Date de publication et mention de la délivrance du brevet: **18.06.2014**
(21) Numéro de dépôt: 10714274.7
(22) Date de dépôt: 16.04.2010
(51) Int. Cl.: B01F 1/00, B01F 5/04, A61M 1/16

(54) **PROCÉDÉ ET DISPOSITIF DE MISE EN SOLUTION D'UN CONCENTRÉ SOLIDE**
Verfahren und Vorrichtung zum Lösen eines festen Konzentrats
METHOD AND DEVICE FOR DISSOLVING A SOLID CONCENTRATE

(30) Priorité: 23.04.2009 FR 0952649
(43) Date de publication de la demande: 29.02.2012
(73) Titulaire: Fresenius Medical Care Deutschland GmbH, 61352 Bad Homburg (DE)
(72) Inventeur: GASTAUER, Paul, Hongkong (CN); LAFFAY, Philippe, F-69110 Sainte Foy Les Lyon (FR); LUAIRE, Benoît, 69210 Sourcieux les Mines (FR)
(74) Mandataire: Vièl, Frédérique
(86) Numéro de dépôt international: PCT/EP2010/055071
(87) Numéro de publication internationale: WO 2010/121972

(56) Documents cités:
- EP-A2- 0 917 881
- WO-A1-2005/053413
- GB-A- 1 113 675
- GB-A- 2 025 787
- US-B1- 6 221 321

## Description

L'invention concerne un dispositif pour la mise en solution d'un concentré à l'état pulvérulent, composé d'une conduite d'alimentation en liquide dont la première extrémité est munie de moyens pour la raccorder à une source de liquide sous pression et dont la deuxième extrémité est raccordée à un point de jonction, d'une conduite d'extraction dont la première extrémité est raccordée au point de jonction et qui est munie d'une valve et d'une conduite de transfert dont la première extrémité est raccordée au point de jonction et dont la deuxième extrémité est munie de moyens pour la raccorder à un récipient contenant le concentré à solubiliser. L'invention concerne également un procédé pour la mise en solution d'un concentré à l'état pulvérulent contenu dans un récipient muni d'une seule ouverture, lequel procédé consiste à faire pénétrer un liquide provenant d'une source de liquide sous pression dans le récipient par une ouverture puis à extraire la solution enrichie en produit via la même ouverture par aspiration.

Il est courant dans les procédés d'hémodialyse de réaliser les solutions nécessaires directement avant le traitement, voire même durant le traitement. Ces solutions sont obtenues en solubilisant un produit solide, appelé concentré solide, avec un liquide approprié, en général de l'eau obtenue par osmose inverse (eau RO). Le concentré solide est en général une poudre soluble ou un mélange de poudres solubles. Le concentré solide est vendu dans des poches ou des cartouches munies de raccords permettant leur raccordement aux machines d'hémodialyse. L'eau est introduite dans le récipient et se charge en sel jusqu'à atteindre la saturation. La solution saturée est alors extraite du récipient et transmise à un dispositif de dilution dans lequel la solution saturée est diluée avec de l'eau (ou le liquide approprié) jusqu'à la concentration souhaitée.

Dans un premier type de machine, l'eau pénètre par un premier raccord et sort par un second raccord. Ce type de dispositif permet d'obtenir la solution saturée en continu. Un tel dispositif est décrit par exemple dans le document EP 0 278 100 A2 ou dans le document US 7,077,956 B2. Dans ce dernier cas, le dispositif est muni d'un tube de Venturi pour siphonner la cartouche à la fin de la procédure de dialyse. L'entrée principale du tube de Venturi est reliée à la source d'eau via une conduite de décharge munie d'une première valve. La prise de vide du tube de Venturi est raccordée à une deuxième conduite de décharge reliée à la sortie de la cartouche. Une deuxième valve permet de fermer cette deuxième conduite de décharge. Lors de la procédure de mise en solution, les deux conduites de décharge sont fermées et l'eau pénètre dans la cartouche par le haut tandis que la solution saturée est aspirée par une pompe par le bas de la cartouche. À la fin de la procédure, la pompe est éteinte et les deux valves de décharge sont ouvertes. L'eau provenant de la source d'eau traverse le tube de Venturi en aspirant le reste de solution contenue dans la cartouche. La sortie du tube de Venturi débouche dans un réservoir. Ce dispositif de décharge ne permet pas la mise en solution du concentré solide.

Dans un second type de machine, l'extraction de la solution saturée est réalisée par le même raccord que l'alimentation en eau. Un tel dispositif est connu par exemple du document EP 0 917 881 A2, décrivant un procédé selon le préambule de la revendication 1. Ce dispositif permet de réaliser des charges successives de solution saturée, jusqu'à épuisement du concentré solide. Ce dispositif est cependant particulièrement compliqué. Il comprend une conduite d'alimentation en eau débouchant sur un point de jonction d'où partent d'une part une conduite d'extraction et d'autre part une conduite de transfert. Chacune de ces conduites est munie d'une valve. La conduite d'alimentation est raccordée à une source d'eau, tandis que la conduite d'extraction mène à un dispositif de dilution. Une conduite supplémentaire relie la source d'eau au dispositif de dilution. Dans un mode de réalisation privilégié de l'invention, la conduite d'alimentation est reliée à un réseau d'alimentation sous pression. Dans un premier temps, la valve de la conduite d'extraction est fermée tandis que les deux autres valves sont ouvertes. L'eau sous pression passe donc à travers la conduite d'alimentation, le point de jonction et la conduite de transfert avant de pénétrer dans le récipient. Un capteur détermine le moment où le récipient est plein et donc la fin de l'étape de remplissage. L'unité de commande ferme alors la valve de la conduite d'alimentation et ouvre la valve de la conduite d'extraction. Une pompe située dans l'unité de dilution est mise en marche et la solution saturée contenue dans le récipient est aspirée. Si la conduite d'alimentation n'est pas reliée à une source d'eau sous pression, il est alors nécessaire de prévoir une pompe. Le document prévoit d'utiliser une pompe à piston située dans une quatrième conduite débouchant sur le point de jonction. La valve située dans la conduite d'alimentation et la valve située dans la conduite de transfert sont alors fermées alternativement pour permettre dans un premier temps le remplissage du piston avec l'eau puis l'injection de l'eau dans le récipient. Ce dispositif est donc particulièrement compliqué et nécessite au moins deux valves et une pompe pour aspirer le liquide saturé.

Le document WO 2005/053413 A1 divulgue un appareil selon le préambule de la revendication 5 permettant de traiter des produits alimentaires avec un liquide. Il comporte un contenant pour liquide, une unité de traitement pour le produit alimentaire et un conduit d'alimentation raccordant le contenant pour liquide à l'unité de traitement par l'intermédiaire d'une pompe. Cet appareil comprend un moyen d'alimentation permettant d'ajouter des additifs. Ces additifs sont ajoutés au liquide dans une zone de turbulence provoquée par une restriction située en amont du point de mélange.

L'objectif de l'invention est donc de simplifier le dispositif présenté précédemment pour en faciliter la commande et en diminuer le nombre de pièces et le risque de dysfonctionnement.

Cet objectif est atteint conformément à l'invention du fait que le point de jonction est placé dans l'étranglement d'un tube de Venturi, la conduite d'alimentation étant reliée à la chambre convergente, la conduite d'extraction à la chambre divergente et la conduite de transfert à la prise de vide partant de l'étranglement du tube de Venturi, la valve placée dans la conduite d'extraction pouvant être ouverte ou fermée. De plus, le dispositif est muni de moyens de commande pour commander l'ouverture ou la fermeture de la valve en fonction du taux de remplissage du récipient. Ici, seule la conduite d'extraction est munie d'une valve. Ainsi, lors du raccord de la conduite d'alimentation en liquide sous pression, si la valve est fermée, le liquide pénètre dans le tube de Venturi par la chambre de convergence (entrée principale du tube de Venturi) et, ne pouvant ressortir par la chambre de divergence (sortie du tube de Venturi) en raison de la valve fermée, ressort par la prise de vide partant transversalement depuis l'étranglement. Il traverse alors la conduite de transfert et pénètre dans le récipient où il se charge à saturation du produit à dissoudre. Lorsque la valve est ouverte, le liquide en provenance de la source pénètre dans la chambre de convergence, traverse l'étranglement et ressort par la chambre de divergence provoquant une dépression dans la prise de vide. Le liquide saturé contenu dans le récipient est alors aspiré et entraîné par le liquide traversant le venturi. Lorsque le récipient est vide de liquide, il reste du concentré solide. La valve est refermée et le cycle est recommencé. Le dispositif conforme à l'invention ne nécessite qu'une seule valve. Avec le tube de venturi, qui ne nécessite aucune alimentation en électricité ni aucun dispositif de commande, il n'est plus nécessaire de prévoir une pompe spécifiquement destinée à l'aspiration du liquide saturé.

Lorsque le dispositif est destiné à être relié à une source de liquide sous pression atmosphérique, il est préférable de placer une pompe dans la conduite d'alimentation. Ainsi, le liquide pourra être prélevé d'un réservoir.

Afin de permettre une automatisation du procédé, il est préférable de prévoir des moyens pour mesurer le taux de remplissage en liquide du récipient. On pourra alors prévoir que les moyens de commandes soient des moyens pour commander l'ouverture ou la fermeture de la valve en fonction d'un signal émis par les moyens de mesure du taux de remplissage en liquide du récipient. Dans un mode de réalisation privilégié de l'invention, les moyens de mesure du taux de remplissage en liquide du récipient sont constitués par un capteur de pression disposé dans la conduite de transfert. Ce capteur de pression permettra de déterminer d'une part le moment où le récipient sera plein de liquide lors de la première partie du cycle et d'autre part le moment où tout le liquide aura été aspiré lors de la deuxième partie du cycle.

Dans le procédé conforme à l'invention, le liquide est introduit dans le récipient en traversant un tube de Venturi, le liquide pénétrant par la chambre convergente dudit tube et en ressortant par la prise de vide dudit tube, avant de pénétrer dans le récipient, et en ce que l'aspiration de la solution enrichie est obtenue en faisant circuler le liquide sous pression dans le tube de Venturi entre la chambre convergente et la chambre divergente du tube de Venturi. De plus, il est prévu pour provoquer l'aspiration de la solution enrichie, de commander la circulation du liquide à travers le tube de Venturi en fonction de l'état de remplissage du récipient.

Afin d'automatiser le procédé, il est prévu de déterminer la fin du remplissage du récipient avec du liquide et/ou la fin de l'aspiration de la solution enrichie. Cette détermination est préférence réalisée en mesurant la pression entre la prise de vide du tube de Venturi et le récipient. La circulation du liquide à travers le tube de Venturi peut être déclenchée lorsque le récipient est rempli avec le liquide et/ou la circulation du liquide à travers le tube de Venturi est arrêtée lorsque l'aspiration de la solution enrichie est achevée.

L'invention est décrite plus en détail ci-dessous à l'aide des figures suivantes qui montrent :

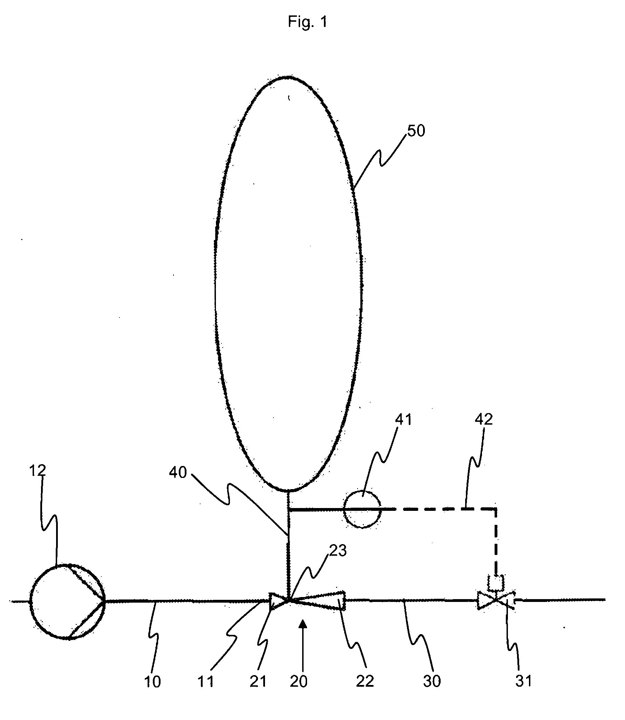

- Figure 1 :: une vue schématique du dispositif ;
- Figure 2 :: la même vue lors de la phase de remplissage ; et
- Figure 3 :: la même vue lors de la phase d'extraction.

Le dispositif de l'invention est destiné en premier lieu à une machine d'hémodialyse. Il peut cependant être utilisé pour tout autre type de machine de mise en solution. Dans la suite, il sera en général fait référence à de l'eau, notamment de l'eau RO. Il va de soi que tout autre liquide approprié pourra être utilisé en fonction de l'usage prévu.

Dans le cas de l'hémodialyse, la solution extraite du récipient doit être une solution saturée. Il est cependant possible que pour d'autres applications une solution non saturée soit souhaitable. C'est pourquoi il est fait référence à une solution enrichie en produit, cet enrichissement pouvant aller jusqu'à la saturation.

Le dispositif est destiné à être raccordé d'une part à une source de liquide sous pression ou non et d'autre part à un récipient contenant le concentré solide. Si la source de liquide n'est pas sous pression, le dispositif est muni d'une pompe de sorte qu'à la sortie de la pompe on se trouve en présence d'une source de liquide sous pression. La sortie du dispositif est destinée à être raccordée par exemple à une unité de dilution de la machine d'hémodialyse.

Le dispositif est constitué d'une conduite d'alimentation (10) en eau sous pression dont l'une des extrémités (11) est raccordée à l'entrée (21) d'un tube de Venturi (20). La deuxième extrémité est munie de moyens pour la connecter à une source de liquide. La sortie (22) du tube de Venturi (20) est raccordée à la première extrémité d'une conduite d'extraction (30) dans laquelle est insérée une valve (31) pouvant être ouverte ou fermée.

La prise de vide (23) du tube de Venturi (20) est raccordée à la première extrémité d'une conduite de transfert (40). La deuxième extrémité de la conduite de transfert (40) est munie de moyens pour la connecter au raccord d'un récipient (50) contenant un concentré solide.

Afin de permettre une automatisation du procédé de solubilisation, il est préférable de prévoir des moyens pour mesurer le taux de remplissage en liquide du récipient lors du remplissage de l'eau RO et lors de l'aspiration du liquide saturé. De même, il est préférable de prévoir des moyens de commande pour commander l'ouverture ou la fermeture de la valve en fonction de paramètres prédéfinis, notamment en fonction du taux de remplissage du récipient. Dans l'exemple présenté ici, les moyens pour mesurer le taux de remplissage en liquide du récipient sont constitués par un capteur de pression (41) placé dans la conduite de transfert (40). Ces moyens de mesure servent à commander la valve (31) via la ligne de commande (42). Ils fournissent le signal nécessaire aux moyens de commande de l'ouverture ou la fermeture de la valve.

L'alimentation en eau du dispositif peut se faire à l'aide d'un réseau de distribution présentant une certaine pression, par exemple 1,3 bar. Il est également possible de faire appel à une source d'eau telle qu'une poche d'eau RO et d'utiliser une pompe (12) placée dans la conduite d'alimentation (10).

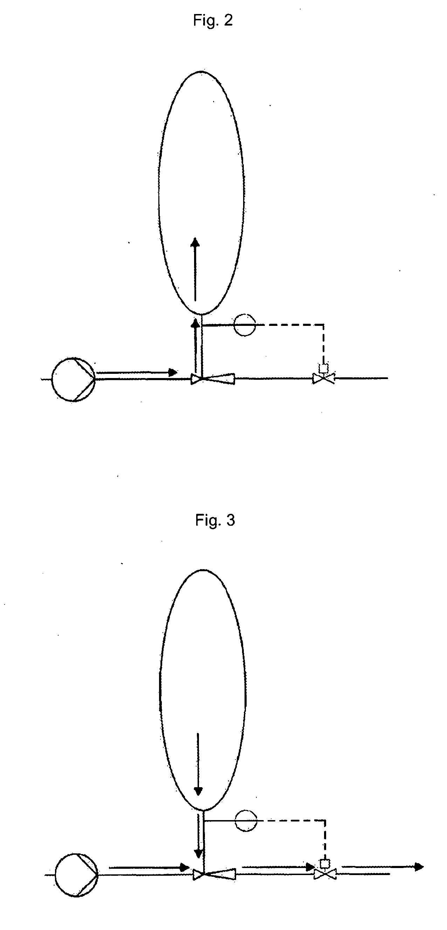

Le dispositif de l'invention fonctionne de la façon suivante. Dans une première étape représentée à la figure 2, l'eau RO est introduite dans le récipient (50). Pour cela, la valve (31) est fermée et la pompe (12) mise en marche. L'eau traverse la conduite d'alimentation (10), entre dans le tube de Venturi (20) par son entrée (21) et en ressort par la prise de vide (23), traverse la conduite de transfert (40) et pénètre dans le récipient (50). Selon le modèle, cette première étape est considérée comme achevée après un certain laps de temps considéré comme suffisant pour remplir le récipient (50). Dans le cas de l'exemple représenté sur les figures, cette première étape est achevée lorsque la pression mesurée par le capteur (41) dans la conduite de transfert (40) a atteint une première valeur seuil. Durant cette étape, l'eau passe au travers du concentré solide se trouvant au fond du récipient et se charge jusqu'à saturation de ce produit.

Une fois cette première étape achevée, la deuxième étape est réalisée telle que représentée sur la figure 3. La valve (31) est ouverte permettant ainsi à l'eau sous pression provenant de la conduite d'alimentation (10) de traverser le tube de Venturi (20). La circulation de l'eau dans le tube de Venturi (20) provoque une dépression au niveau de la prise de vide (23). La solution saturée se trouvant dans le récipient est alors aspirée rapidement et pratiquement complètement via la prise de vide (23) et se trouve ainsi mélangée à l'eau RO provenant de l'entrée (21) du tube de Venturi (20). Dès que le récipient est vide, la valve (31) est refermée et le cycle recommence. La fin de l'aspiration du liquide saturé est mesurée par exemple à l'aide du capteur de pression (41) qui, dès que la pression dans la conduite de transfert (40) passe en dessous d'une deuxième valeur seuil, commande la fermeture de la valve (31).

Ce dispositif est beaucoup plus simple que celui des dispositifs de l'état de la technique. Il ne nécessite qu'une seule valve qui peut être automatisée grâce à des moyens de mesure du taux remplissage en liquide du récipient. Si le dispositif est raccordé à un réseau d'alimentation en eau présentant une pression suffisante, il n'est pas nécessaire de prévoir de pompe. Si l'eau d'alimentation n'est pas sous pression, il suffit d'une seule pompe.

## Revendications

1. Procédé pour la mise en solution d'un concentré à l'état pulvérulent contenu dans un récipient (50) muni d'une seule ouverture, lequel procédé consiste à faire pénétrer un liquide provenant d'une source de liquide dans le récipient (50) par une ouverture puis à extraire la solution enrichie via la même ouverture par aspiration, **caractérisé en ce que** le liquide est introduit dans le récipient (50) en traversant un tube de Venturi (20), le liquide pénétrant par la chambre convergente (21) dudit tube et en ressortant par la prise de vide (23) dudit tube, avant de pénétrer dans le récipient (50), et **en ce que** l'aspiration de la solution enrichie est obtenue en faisant circuler le liquide sous pression dans le tube de Venturi (20) entre la chambre convergente (21) et la chambre divergente (22), et **en ce que** la circulation du liquide à travers le tube de Venturi (20) pour provoquer l'aspiration de la solution enrichie est commandée en fonction de l'état de remplissage du récipient.

2. Procédé selon la revendication précédente, **caractérisé en ce que** la fin du remplissage du récipient (50) avec du liquide et/ou la fin de l'aspiration de la solution enrichie sont déterminées.

3. Procédé selon la revendication précédente, **caractérisé en ce que** la fin du remplissage du récipient (50) avec du liquide et/ou la fin de l'aspiration de la solution enrichie sont déterminées en mesurant la pression entre la prise de vide (23) du tube de Venturi et le récipient (50).

4. Procédé selon l'une des revendications précédentes, **caractérisé en ce que** la circulation du liquide à travers le tube de Venturi (20) est déclenchée lorsque le récipient est rempli avec le liquide et/ou la circulation du liquide à travers le tube de Venturi (20) est arrêtée lorsque l'aspiration de la solution enrichie est achevée.

5. Dispositif pour la mise en oeuvre du procédé selon l'une des revendications précédentes afin de mettre en solution un concentré à l'état pulvérulent contenu dans un récipient muni d'une seule ouverture, lequel dispositif est composé
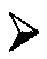 d'une conduite d'alimentation (10) en liquide dont la première extrémité est munie de moyens pour la raccorder à une source de liquide et dont la deuxième extrémité est raccordée à un point de jonction ;
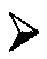 d'une conduite d'extraction (30) dont la première extrémité est raccordée au point de jonction et qui est munie d'une valve (31) ; et
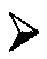 d'une conduite de transfert (40) dont la première extrémité est raccordée au point de jonction et dont la deuxième extrémité est munie de moyens pour la raccorder à un récipient (50) contenant le concentré à solubiliser
**caractérisé en ce que** le point de jonction est placé dans l'étranglement d'un tube de Venturi (20), la conduite d'alimentation (10) étant reliée à la chambre convergente (21), la conduite d'extraction (30) à la chambre divergente (22) et la conduite de transfert (40) à l'étranglement (23) du tube de Venturi (20), et **en ce que** la valve (31) placée dans la conduite d'extraction (30) peut être ouverte ou fermée, le dispositif étant muni de moyens de commande pour commander l'ouverture ou la fermeture de la valve en fonction du taux de remplissage du récipient.

6. Dispositif selon la revendication précédente, **caractérisé en ce qu**'une pompe (12) est placée dans la conduite d'alimentation (10).

7. Dispositif selon la revendication 5 ou 6, **caractérisé en ce que** des moyens sont prévus pour mesurer le taux de remplissage en liquide du récipient (50).

8. Dispositif selon la revendication 7, **caractérisé en ce que** les moyens de commande sont des moyens pour commander l'ouverture ou la fermeture de la valve (31) en fonction d'un signal émis par les moyens de mesure du taux de remplissage en liquide du récipient (50).

9. Dispositif selon la revendication 7 ou 8, **caractérisé en ce que** les moyens de mesure du taux de remplissage en liquide du récipient (50) sont constitués par un capteur de pression (41) disposé dans la conduite de transfert (40).

## Patentansprüche

1. Verfahren zum Auflösen eines Konzentrats in pulverförmigem Zustand, das in einem Behälter (50) enthalten ist, der mit einer einzigen Öffnung versehen ist, wobei das Verfahren darin besteht, eine von einer Flüssigkeitsquelle stammende Flüssigkeit in einen Behälter (50) durch eine Öffnung eintreten zu lassen, dann die angereicherte Lösung durch die gleiche Öffnung durch Ansaugen abzuziehen, **dadurch gekennzeichnet, dass** die Flüssigkeit in den Behälter (50) unter Durchströmen eines Venturirohrs (20) eingeleitet wird, wobei die Flüssigkeit durch die konvergente Kammer (21) des Rohrs eintritt und durch den Vakuumanschluss (23) des Rohrs wieder austritt, bevor sie in den Behälter (50) eintritt, und dass das Ansaugen der angereicherten Lösung dadurch erreicht wird, dass die Flüssigkeit unter Druck im Venturirohr (20) zwischen der konvergenten Kammer (21) und der divergenten Kammer (22) zirkuliert, und dass die Zirkulation der Flüssigkeit durch das Venturirohr (20) hindurch zum Ansaugen der angereicherten Lösung in Abhängigkeit des Füllungsgrades des Behälters gesteuert wird.

2. Verfahren nach dem vorangehenden Anspruch, **dadurch gekennzeichnet, dass** das Ende der Befüllung des Behälters (50) mit der Flüssigkeit und/oder das Ende des Ansaugens der angereicherten Lösung bestimmt wird.

3. Verfahren nach dem vorangehenden Anspruch, **dadurch gekennzeichnet, dass** das Ende der Befüllung des Behälters (50) mit der Flüssigkeit und/oder das Ende des Ansaugens der angereicherten Lösung durch Messen des Drucks zwischen dem Vakuumanschluss (23) des Venturirohrs und dem Behälter (50) bestimmt wird.

4. Verfahren nach einem der vorangehenden Ansprüche, **dadurch gekennzeichnet, dass** die Zirkulation durch das Venturirohr (20) hindurch in Gang gebracht wird, wenn der Behälter mit der Flüssigkeit gefüllt ist und/oder die Zirkulation durch das Venturirohr (20) hindurch gestoppt wird, wenn das Ansaugen der angereicherten Lösung beendet ist.

5. Vorrichtung für die Durchführung des Verfahrens nach einem der vorangehenden Ansprüche, um ein Konzentrat in pulverförmigem Zustand aufzulösen, das in einem Behälter, der mit einer einzigen Öffnung versehen ist, enthalten ist, wobei die Vorrichtung besteht aus
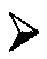 einer Zuführleitung (10) für Flüssigkeit, deren erstes Ende mit Mitteln versehen ist, um sie an eine Flüssigkeitsquelle anzuschließen und deren zweites Ende an einer Verbindungsstelle angeschlossen ist;
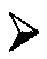 einer Auslassleitung (30), deren erstes Ende an der Verbindungsstelle angeschlossen ist und die mit einem Ventil (31) versehen ist; und
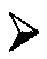 einer Verbindungsleitung (40), deren erstes Ende an der Verbindungsstelle angeschlossen ist und deren zweites Ende mit Mitteln versehen ist, um sie an einen Behälter (50) anzuschließen, der das zu lösende Konzentrat enthält,
**dadurch gekennzeichnet, dass** die Verbindungsstelle in der Verengung eines Venturirohrs (20) angeordnet ist, wobei die Zufuhrleitung (10) mit der konvergenten Kammer (21), die Auslassleitung (30) mit der divergenten Kammer (22) und die Verbindungsleitung (40) mit der Verengung (23) des Venturirohrs (20) verbunden ist und dass das in der Auslassleitung (30) angeordnete Ventil (31) geöffnet oder geschlossen werden kann, wobei die Vorrichtung mit Steuerungsmitteln versehen ist, um das Öffnen oder Schließen des Ventils in Abhängigkeit des Behälterfüllungsgrades zu steuern.

6. Vorrichtung nach dem vorangehenden Anspruch, **dadurch gekennzeichnet, dass** eine Pumpe (12) in der Zuführleitung (10) angeordnet ist.

7. Vorrichtung nach Anspruch 5 oder 6, **dadurch gekennzeichnet, dass** Mittel für das Messen des Flüssigkeitsfüllungsgrades des Behälters (50) vorgesehen sind.

8. Vorrichtung nach Anspruch 7, **dadurch gekennzeichnet, dass** die Steuerungsmittel Mittel sind, um das Öffnen oder Schließen des Ventils (31) in Abhängigkeit eines Signals zu steuern, das von den Messmitteln für den Flüssigkeitsfüllungsgrad des Behälters (50) ausgegeben wird.

9. Vorrichtung nach Anspruch 7 oder 8, **dadurch gekennzeichnet, dass** die Messmittel für den Flüssigkeitsfüllungsgrad des Behälters (50) aus einem Drucksensor (41) bestehen, der in der Verbindungsleitung (40) angeordnet ist.

## Claims

1. Method for putting in solution a concentrate in the powdery state contained in a receptacle (50) provided with a single opening, the said method consisting of causing a liquid coming from a source of liquid to enter the receptacle (50) through an opening and then extracting the enriched solution via the same opening by suction, **characterised in that** the liquid is introduced into the receptacle (50) by passing through a venturi tube (20), the liquid entering through the convergent chamber (21) of the said tube and leaving through the vacuum tapping (23) of the said tube, before entering the receptacle (50), and **in that** the suction of the enriched solution is obtained by causing the pressurised liquid to flow in the venturi tube (20) between the convergent chamber (21) and the divergent chamber (22), and **in that** the flow of the liquid through the venturi tube (20) to cause the suction of the enriched solution is controlled according to the degree of filling of the receptacle.

2. Method according to the preceding claim, **characterised in that** the end of the filling of the receptacle (50) with liquid and/or the end of the suction of the enriched solution are determined.

3. Method according to the preceding claim, **characterised in that** the end of the filling the receptacle (50) with liquid and/or the end of the suction of the enriched solution are determined by measuring the pressure between the vacuum tapping (23) of the venturi tube and the receptacle (50).

4. Method according to one of the preceding claims, **characterised in that** the flow of liquid through the venturi tube (20) is triggered when the receptacle has been filled with the liquid and/or the flow of the liquid through the venturi tube (20) is stopped when the suction of the enriched solution is complete.

5. Device for carrying out the method according to one of the preceding claims for putting in solution a concentrate in the powdery state contained in a receptacle provided with a single opening, said device being composed of:
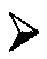 a liquid supply pipe (10) the first end of which is provided with means for connecting it to a liquid source and a second end of which is connected to a junction point;
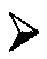 an extraction pipe (30) the first end of which is connected to the junction point and which is provided with a valve (31); and
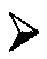 a transfer pipe (40) the first end of which is connected to the junction point and the second end of which is provided with means for connecting it to a receptacle (50) containing the concentrate to be solubilised,
**characterised in that** the junction point is placed in the constriction of a venturi tube (20), the supply pipe (10) being connected to the convergent chamber (21), the extraction pipe (30) to the divergent chamber (22) and the transfer pipe (40) to the constriction (23) of the venturi tube (20) and **in that** the valve (31) placed in the extraction pipe (30) may be open or closed, wherein the device is provided with controlling means for controlling the opening or closing of the valve according to the degree of filling of the receptacle.

6. Device according to the preceding claim, **characterised in that** a pump (12) is placed in the supply pipe (10).

7. Device according to claim 5 or 6, **characterised in that** means are provided for measuring the degree of filling of the receptacle (50) with liquid.

8. Device according to claim 7, **characterised in that** the controlling means are means for controlling the opening or closing of the valve (31) according to a signal emitted by the means of measuring the degree of filling of the receptacle (50) with liquid.

9. Device according to claim 7 or 8, **characterised in that** the means of measuring the degree of filling of the receptacle (50) with liquid are formed by a pressure sensor (41) disposed in the transfer pipe (40).
